# EUROPEAN PATENT SPECIFICATION

(11) **EP 3 632 421 B1**
(45) Date of publication and mention of the grant of the patent: **13.08.2025**
(21) Application number: 18809564.0
(22) Date of filing: 17.05.2018
(51) Int. Cl.: A61K 31/19, A23L 33/10, A61P 25/28, A23L 2/68, A61K 9/00

(54) **ACETIC ACID AND/OR AN ACETIC ACID SALT FOR USE IN THE TREATMENT OF ALZHEIMER-TYPE DEMENTIA**
ESSIGSÄURE UND/ODER EIN ESSIGSÄURESALZ ZUR VERWENDUNG BEI DER BEHANDLUNG VON ALZHEIMER-TYP DEMENZERKRANKUNGEN
ACIDE ACÉTIQUE ET/OU SEL D'ACIDE ACÉTIQUE POUR UTILISATION DANS LE TRAITEMENT DE LA DÉMENCE DE TYPE ALZHEIMER

(30) Priority: 30.05.2017 JP 2017106200
(43) Date of publication of application: 08.04.2020
(73) Proprietor: Morinaga Milk Industry Co., Ltd., Minato-ku Tokyo 105-7122 (JP)
(72) Inventor: KOBAYASHI, Youdai, Zama-shi Kanagawa 252-8583 (JP); KUHARA, Tetsuya, Zama-shi Kanagawa 252-8583 (JP)
(74) Representative: Vossius & Partner Patentanwälte Rechtsanwälte mbB
(86) International application number: PCT/JP2018/019108
(87) International publication number: WO 2018/221244

(56) References cited:
- EP-A1- 2 103 304
- WO-A2-03/105658
- CN-A- 106 174 531
- JP-A- 2002 255 801
- JP-A- 2005 350 391
- JP-A- 2007 070 342
- JP-A- 2008 239 525
- JP-A- 2010 522 216
- JP-A- 2013 060 380
- JP-A- 2016 007 209
- JP-A- H08 188 532
- JP-A- S62 185 018
- HIROAKI KANOUCHI ET AL: "The Brewed Rice Vinegar Kurozu Increases HSPA1A Expression and Ameliorates Cognitive Dysfunction in Aged P8 Mice", PLOS ONE, vol. 11, no. 3, 4 March 2016 (2016-03-04), pages e0150796, XP055764628, DOI: 10.1371/journal.pone.0150796

## Description

### Technical Field

The present technique relates to a composition for improving brain function, for example, a brain function improver and a food or drink composition for improving brain function.

### Background Art

Since all persons of young, middle, or old ages have always expected improvement of brain functions regardless of his/her age or gender, pharmaceuticals, herbal medicines, Chinese medicines, food or drink products, and the like, that are safe and that can be administered for a long period of time, are required.

In particular, with an extended life-span in the coming super-aging society, it is one important social issue to prevent declines in brain functions due to aging or declines in brain functions due to age-related brain diseases to extend the healthy life-span where a human can aggressively act and to suppress a decrease in quality of life (QOL) in his/her lifetime.

Furthermore, prophylaxis, treatment, or improvement of dementia in which declines in learning and memory abilities, among declines in brain functions, are gradually shown and which considerably affects daily life, is an urgent task. Besides, a decline in brain function may occur by brain damage due to a traffic accident, a cerebrovascular disorder, or the like, and symptoms from such causes are called higher brain dysfunction.

Dementia can be roughly divided into vascular dementia and Alzheimer's-type dementia.

Vascular dementia is dementia caused by necrotic nerve cells due to a cerebrovascular disorder, such as brain infarction or subarachnoid hemorrhage.

Alzheimer's-type dementia includes early-onset dementia and age-related dementia. Declines in cognitive functions and an alteration in personality are main symptoms of Alzheimer's-type dementia. Alzheimer's disease is categorized in neurodegenerative diseases which also includes Parkinson's disease and amyotrophic lateral sclerosis (ALS) (NPL 1). The cause of Alzheimer's disease has long been studied and a social need has recently been increasing for etiology and therapeutics of the disease against the background of the frequency thereof.

Alzheimer's disease accounts for 50 to 60% of nerve diseases, and the symptoms are considered to be caused by a protein, β-amyloid, accumulating in the brain. The accumulating β-amyloid breaks normal nerve cells to further cause brain atrophy (NPL 2). In addition, it is reported that the concentration of lactic acid increases in the cerebrospinal fluid of a patient of Alzheimer's disease (NPL 3) .

Possible causes of Alzheimer's disease include an age-related change of the brain, a genetic factor, an environmental factor, and a lifestyle-related factor, but the causes have yet to be fully elucidated.

As a composition for improving brain function, for example, PTL 1 disclose a composition for improving brain function that contains as an active ingredient an extract mainly containing a polyphenol and obtained by extracting leaves of Ligustrum purpurascens Y. C. Yang with water, an organic solvent alone, or a mixture thereof, the extract having an antioxidative activity and/or aldose reductase inhibitory activity.

However, there have yet been few findings about a compound or the like that improves a brain function and thus further exploration and research are being promoted about compounds having a brain function improving effect.

PTL 2 discloses a method of treating hypertension.

PTL 3 discloses an orally administered composition comprising a magnesium-containing compound (MCC). The active ingredient is magnesium and the concrete compound used in the experiments is magnesium threonate.

PTL 4 discloses a pharmaceutical composition which contains lithium as the active ingredient.

PTL 5 discloses an Alzheimer's diseases preventing and treating agent containing a pyroligneous liquor as an active ingredient.

PTL 6 discloses a composition for improving brain function comprising vinegar. The active ingredient to which the therapeutic effect is attributed is an alkaline stable lipid, such as N-acyl sphinganine and 2' - hydroxypalmitoyl-sphinganine.

PTL 7 discloses a food product comprising black vinegar.

PTL 8 discloses use of odorant compounds for treating Alzheimer's diseases, and acetic acid are mentioned as odorant compounds.

PTL 9 discloses an agent for preventing and improving cerebrovascular dementia comprises citric acid and / or isocitric acid as an active ingredient.

PTL 10 discloses the use of chitin as a health food, primarily to treat diabetes. Acetic acid is used as a solvent of chitin, while no therapeutic effect is attributed to acetic acid as such.

PTL 11 discloses use of a food composition comprising black vinegar for improving brain function, cognitive impairment, dementia, and Alzheimer's disease. The black vinegar mash powder is used as the active ingredient in which the acetic acid has been removed from black vinegar.

NPL 7 discloses that brewed rice vinegar Kurozu ameliorates cognitive dysfunction and attributes this to an antioxidant effect of concentrated Kurozu.

PTL 12 discloses a composition that can comprise vinegar for ameliorating a cerebral function. The active ingredient to which the therapeutic effect is attributed is an alkali-stable lipid, i.e. N-acylsphinganine. The lipid is prepared from an acetic acid bacterial strain by chloroform-methanol extraction and subsequent drying.

PTL 13 discloses that "prevention of Alzheimer's dementia, confirmed the action to smooth the flow of action and blood flow to widen the blood vessels" and "improve early stages of senile dementia Alzheimer's"; see para. [0006] and last para. of PTL 13. No experimental data are provided to corroborate this. PTL 13 further suggests preventing and treating thrombosis and arterial hardening by injecting diluted vinegar into the body; see last para. of PTL 13. The rationale provided is that the vinegar might have such an effect by promoting "blood flow to widen bold vessels". However, also here no such data are provided for vinegar, let alone that the "promotion of blood flow" has an effect on dementia.

### Citation List

### Patent Literature

PTL 1: JP-A-2010-163460
PTL 2: JP2002-255801
PTL 3: JP2010-522216
PTL 4: JPS62-185018
PTL 5: JP2005-350391
PTL 6: JP2007-070342
PTL 7: JP2016-007209
PTL 8: WO 03/105658
PTL 9: JP2008-239525
PTL 10: CN106174531
PTL 11: JP2013-060380
PTL 12: EP2103304
PTL 13: JP H08/188532

### Non-patent Literature

NPL 1: Igaku no Ayumi (Journal of Clinical and Experimental Medicine), Vol. 247, No. 5, 465-471, 2013.
NPL 2: Selkoe, D.J., Physiological Reviews, 81, 741-766, 2001
NPL 3: Liguori et al., Journal of Neurological and Neurosurgical Psychiatry, 86(6), 655-659, 2015
NPL 4: Nippon Eiyo Syokuryo Gakukaishi (Journal of Japan Society of Nutrition and Food Science), Vol. 67, No. 4, 171-176, 2014.
NPL 5: Furusawa et al., Nature 504, 446-450, 2013
NPL 6: Takeda et al., Brain Research, 1280, 137-147, 2009
NPL 7: Kanouchi, Hiroaki, et al. "The brewed rice vinegar Kurozu increases HSPA1A expression and ameliorates cognitive dysfunction in aged P8 mice." PLoS One 11.3 (2016): e0150796.

### Summary

In view of the above situation, a primary object of the present technique is to provide a composition for improving brain function (for example, a brain function improver or a food or drink composition for improving brain function).

Then, as a result of extensive studies, the present inventors surprisingly found that when acetic acid and/or an acetic acid salt was orally taken in a cognitive function determination model, decline in cognitive function was able to be suppressed. Based on the result, the present inventors have found that acetic acid and/or an acetic acid salt, which is safe, can improve brain function.

NPL 4 discloses that acetic acid is used as a living body fuel, that acetic acid activates AMP kinase, and that acetic acid has an obesity suppressing action and a lipid metabolism promoting action. However, direct relationship between acetic acid and brain function improvement is not known.

In addition, NPL 5 discloses the induction of regulatory T-cell differentiation by butyric acid produced by enteric bacteria, but states that no induction of regulatory T-cell differentiation was found by acetic acid. Thus, probiotic actions have very complicated mechanisms. Accordingly, compounds having similar structures do not always serve the same function in the living body (particular in oral intake).

There has yet been no report of brain function improvement by taking acetic acid.

The invention provides a composition comprising acetic acid and/or an acetic acid salt as an active ingredient for use in the treatment of Alzheimer-type dementia. The composition can be for oral intake. The composition can be a food or drink composition. The acetic acid and/or acetic acid salt comprised in the composition can be produced by fermentation.

The invention also provides an acetic acid and/or an acetic acid salt for use in the treatment of Alzheimer-type dementia. The acetic acid and/or the acetic acid salt can be for oral intake. The acetic acid and/or an acetic acid salt can be comprised in a food or drink composition. The acetic acid and/or an acetic acid salt can be produced by fermentation.

Specifically, the present technique includes a composition for use in the treatment of Alzheimer-type dementia (for example, Alzheimer-type dementia improver or food or drink composition for improving Alzheimer-type dementia) containing acetic acid and/or an acetic acid salt as an active ingredient.

The composition for use in the treatment of Alzheimer-type dementia may be for oral intake.

The improving brain function mentioned in the present technique means improvement particularly in a cognitive function (for example, memory, thinking, language, determination, calculation, affect, or the like). Examples of declines in brain functions include a brain function decline by aging, brain dysfunction due to an age-related brain disease, and brain dysfunction due to brain damage.

Examples of symptoms of brain dysfunction include higher brain dysfunction, vascular dementia, and Alzheimer's-type dementia.

Alzheimer's-type dementia includes early-onset dementia and dementia by aging.

Note that acetic acid and/or an acetic acid salt in the present technique may be used for a human or a nonhuman animal (suitably mammal) as a subject, preferably for a human and a pet, more preferably for a human. The technique may be used for a therapeutic purpose.

"Nontherapeutic purpose" has a concept not including any medical practice, that is, therapeutic treatment practices on a human body. Examples thereof include beauty actions and health promotion.

"Improvement" means change for the better of a disease, a symptom, or a condition; prevention or delaying of worsening of a disease, a symptom, or a condition; and reversing, prevention, or delaying of progression of a disease or a symptom.

"Prophylaxis" means prevention or delaying of onset of a disease or a symptom in a subject, or reduction in the risk of a disease or a symptom of a subject. Advantageous Effects

The present technique can provide a composition comprising acetic acid and/or an acetic acid salt as an active ingredient for use in the treatment of Alzheimer-type dementia (for example, Alzheimer-type dementia improver or food or drink composition for improving Alzheimer-type dementia).

### Detailed Description

A suitable embodiment for implementing the present technique will be described below. Note that the embodiment described below is an example of a typical embodiment of the present technique.

The present technique includes a composition comprising acetic acid and/or an acetic acid salt as an active ingredient for use in the treatment of Alzheimer-type dementia. The composition can be a pharmaceutical composition, a food or drink composition, and the like. Among them, an oral composition (for example, an oral Alzheimer-type dementia improver and a food or drink composition for improving Alzheimer-type dementia) is preferred since efficacy can be exhibited in oral intake as shown in Examples described later herein. In oral intake, the present technique can be in the form of tablet, powder, capsule, syrup, oral gel, or liquid diet.

Acetic acid in the present technique is a chemical represented by a chemical formula CH₃COOH. In addition, the "salt" in an acetic acid salt in the present technique is desirably, but not particularly limited to, a component acceptable in pharmaceuticals, food or drink products, or feeds. Since the acidic taste and acidic odor of acetic acid can be reduced in a salt, the form of salt is preferred.

Acetic acid and/or an acetic acid salt in the present technique may be a fermentation-based one or a chemically-synthesized one, and may be used in a state of liquid (specifically, crude liquid, diluted liquid, or the like) or solid (specifically, powder, granule, or the like). Note that acetic acid that is released from an acetic acid compound, such as an acetic acid ester derivative, by biodegradation or the like may exhibit the efficacy of the present technique.

Suitable examples of the salts include one or two or more selected from metal salts and amine salts.

Examples of the metal salts include alkali metal salts, alkali earth metal salts, and other metal salts.

Examples of the alkali metal salts include lithium salt, sodium salt, and potassium salt. Examples of the alkali earth metal salts include beryllium salt, magnesium salt, and calcium salt. Examples of other metal salts include metal salts, such as copper(II) salt, iron(II) salt, and zinc salt.

Examples of the amine salts include ammonium salt, a basic amino acid salt (for example, lysine salt, arginine salt, and histidine salt), N,N'-dibenzylethylenediamine salt, choline salt, diethanol amine salt, ethylenediamine salt, and trishydroxymethylaminomethane salt.

The salts may be used alone or in combination of two or more thereof.

Acetic acid and/or an acetic acid salt in the present technique may be produced by fermentation or chemical synthesis.

Examples of commercial products of acetic acid and/or acetic acid salts include a vinegar, acetic acid (also referred to as glacial acetic acid), sodium acetate, calcium acetate, ammonium acetate, lithium acetate, magnesium acetate, and zinc acetate. A mixture of acetic acid and an acetic acid salt may be used, and an example is a mixture of sodium acetate and glacial acetic acid. The commercial products may be used alone or in combination of two or more thereof.

Among them, a product that can be orally taken and can be industry mass-produced is preferred in terms of safety and cost and examples thereof include acetic acid metal salts.

As described later in Examples, acetic acid and/or an acetic acid salt in the present technique has an action to suppress a cognitive function decline. Thus, acetic acid and/or an acetic acid salt in the present technique has an action to improve Alzheimer-type dementia, and has an action of treatment of the various symptoms of Alzheimer's-type dementia.

Acetic acid and/or an acetic acid salt in the present technique can be used as an active ingredient of a composition for use in the treatment Alzheimer's-type dementia. The composition may be used as a preparation, a food or drink composition, and as a feed.

Acetic acid and/or an acetic acid salt in the present technique can be contained as an active ingredient of various such compositions or preparations. In addition, acetic acid and/or an acetic acid salt in the present technique can be used as an active ingredient for a therapeutic use related to Alzheimer's-type dementia.

Acetic acid and/or an acetic acid salt in the present technique can suppress a decline in a brain function, such as a memory ability or a learning ability in relation to Alzheimer's-type dementia and is safe even in the use for a long period of time, and thus can be used as a content of a therapeutic food or drink product for use in the treatment of Alzheimer's-type dementia.

Then, acetic acid and/or an acetic acid salt in the present technique can be used in the treatment of Alzheimer's-type dementia as described above as an active ingredient of a pharmaceutical, a quasi-drug, a skin external agent, a pharmaceutical composition, a food or drink product, a food or drink composition, or the like for a human or an animal. The composition of the present invention may be further incorporated into such a preparation or composition. In addition, acetic acid and/or an acetic acid salt in the present technique can be used for producing various such preparations or compositions.

In the present technique, in addition to acetic acid and/or an acetic acid salt in the present technique, an optional component may be used, as needed, in combination therewith. As such an optional component, any component that is acceptable in pharmaceuticals, food or drink products, feeds, and the like may be appropriately used. Examples of such optional components include sugars, sugar alcohols, polysaccharides, pH modifiers, fatty acid esters, corrigents, fragrances, and excipients.

The preparation or the composition of the present technique can be produced by a known production method. For example, since acetic acid and/or an acetic acid salt in the present technique is easily available in the form of powder or liquid, the preparation or the composition of the present technique can be obtained by mixing acetic acid and/or an acetic acid salt with another raw material component (solid, semi-solid, liquid, etc.) or by impregnating another solid raw material component with acetic acid and/or an acetic acid salt.

The content or the amount used in the present technique is not particularly limited and can be appropriately selected according to the gender, the interval between intakes, or the like.

The content in the present technique is preferably 0.001 to 30% by mass in terms of acetic acid, more preferably 0.005 to 10% by mass, further preferably 0.01 to 6% by mass, and furthermore preferably 0.05 to 5% by mass. From the viewpoint of the efficacy and the easiness of oral intake, the content is preferably 0.01 to 2% by mass, more preferably 0.1 to 1.5% by mass, and further preferably 0.5 to 1% by mass.

A standard amount used in the present technique is preferably 2.5 to 500 mg/kg body weight/day in terms of acetic acid, more preferably 12.5 to 250 mg/kg body weight/day, and further preferably 25 to 150 mg/kg body weight/day.

The interval of administrations or intakes in the present technique is not particularly limited, and the administration or intake may be performed once a day or may be performed by several divided portions.

The present technique is desirably administered or taken for at least 5 days, and more desirably for at least 10 days. In addition, the present technique is desirably administered or taken in a continuous daily manner.

The subject of the present technique is not particularly limited but is desirably a middle-aged or older person in which a decline in a brain function is likely to occur, and more preferably an old person. Middle age refers to approximately the late 30's to early 50's, and old age refers to the late 50's or older. In addition, the present technique is desirably administered prior to the onset of Alzheimer-type dementia.

The manner of administration of the present technique is not particularly limited but examples include oral administration or oral intake, nasal administration, sublingual administration, instillation administration, inhalation administration, transrectal administration, injection (intravenous administration, intramuscular administration, subcutaneous administration), and percutaneous administration. Among them, oral administration or oral intake is more preferred since the compound of the present technique is highly safe and such an administration method is simple and convenient.

When acetic acid and/or an acetic acid salt in the present technique is used in a pharmaceutical, the pharmaceutical may be orally or parenterally administered, and oral administration and an administration manner acting on a mucosa are preferred. Examples of parenteral administrations include injection (blood, skin, muscle, etc.), intrarectal administration, and inhalation. Examples of dosage forms for oral administration include tablet, capsule, troche, syrup, granule, powder, and ointment.

In addition, in formulation into a preparation, in addition to acetic acid and/or an acetic acid salt, components ordinarily used in formulation into a preparation, such as an excipient, a pH modifier, a colorant, and a flavoring agent, can be used. Furthermore, a component having an effect of prophylaxis or treatment of a known or possible future disease can be used together according to the purpose.

Furthermore, according to the administration method, the composition may be appropriately formulated into a desired dosage form. For example, in cases of oral administration, the composition can be formulated into solid preparations, such as a powder, granules, tablets, and capsules; and liquid preparations, such as a solution, a syrup, a suspension, and an emulsion. In cases of parenteral administration, the composition can be formulated into suppositories, a spray, an ointment, patches, and an injection.

In addition, the formulation into a preparation can be appropriately achieved by a known method according to the dosage form. In formulation into a preparation, a fraction from separation or purification thereof alone may be formulated into a preparation, or may be formulated into a preparation with a preparation carrier appropriately incorporated.

As the preparation carrier, according to the dosage form, various organic or inorganic carriers can be used. In cases of solid preparations, examples of such carriers include an excipient, a binder, a disintegrator, a lubricant, a stabilizer, and a corrigent.

Examples of excipients include sugar derivatives, such as lactose, sucrose, glucose, mannitol, and sorbitol; starch derivatives, such as corn starch, potato starch, α-starch, dextrin, and carboxymethyl starch; cellulose derivatives, such as crystalline cellulose, hydroxypropylcellulose, hydroxypropylmethylcellulose, carboxymethylcellulose, and carboxymethylcellulose calcium; Arabic rubber; dextran; pullulan; silicate derivatives, such as light anhydrous silicic acid, synthetic aluminum silicate, and magnesium aluminometasilicate; a phosphate derivative, such as calcium phosphate; a carbonate derivative, such as calcium carbonate; and a sulfate derivative, such as calcium sulfate.

Examples of binders include: in addition to the above excipients, gelatin; polyvinylpyrrolidone; and macrogol.

Examples of disintegrators include, in addition to the above excipients, chemically-modified starch or cellulose derivatives, such as croscarmellose sodium, carboxymethylstarch sodium, and crosslinked polyvinyl pyrrolidone.

Examples of lubricants include: talc; stearic acid; metal stearates, such as calcium stearate and magnesium stearate; colloidal silica; waxes, such as veecum and spermaceti; boric acid; glycols; carboxylic acids, such as fumaric acid and adipic acid; a sodium carboxylate, such as sodium benzoate; a sulfuric acid salt, such as sodium sulfate; Leucine; lauryl sulfate salts, such as sodium lauryl sulfate and magnesium lauryl sulfate; silicic acid compounds, such as anhydrous silicic acid and silicic acid hydrate; and starch derivatives.

Examples of stabilizers include: p-oxybenzoic acid esters, such as methylparaben and propylparaben; alcohols, such as chlorobutanol, benzyl alcohol, and phenylethyl alcohol; benzalkonium chloride; acetic anhydride; and sorbic acid.

Examples of corrigents include a sweetener, an acidulant, and a fragrance.

Note that examples of carriers used in the case of liquid preparations for oral administration include a solvent, such as water, and a corrigent.

When acetic acid and/or an acetic acid salt in the present technique is used as a food or drink product for a human or an animal, acetic acid and/or an acetic acid salt can be added to a known food or drink product or can be mixed in raw materials of a food or drink product to produce a new food or drink product. It is also possible to further incorporate acetic acid and/or an acetic acid salt in the present technique into raw materials of an ordinary food or drink product to thereby additionally achieve the efficacy of the present technique.

The food or drink product may be in any form of liquid, paste, solid, and powder, and examples thereof include tablet confections, liquid diets, feeds (including feeds for pets), wheat flour products, ready-to-eat foods, processed agricultural products, processed marine products, processed livestock products, milk or dairy products, oils or fats, basic seasonings, combined seasonings or foods, frozen foods, confections, drinks, and other commercial products.

Examples of wheat flour products include bread, macaroni, spaghetti, noodles, cake mixes, powder for deep-fried foods, and bread crumbs.

Examples of ready-to eat foods include ready-to-eat noodles, cup noodles, retort or prepared foods, prepared canned foods, foods for microwave cooking, ready-to-eat soups or stews, ready-to-eat miso soups or clear soups, canned soups, freeze-dried foods, and other ready-to-eat foods.

Examples of processed agricultural products include canned agricultural products, canned fruits, jams or marmalades, pickles, cooked beans, dried agricultural products, and cereals (processed grains).

Examples of processed marine products include canned marine products, fish meat hams or sausages, marine paste products, marine delicacies, and foods boiled in soy.

Examples of processed livestock products include canned or paste livestock products and livestock meat hams or sausages.

Examples of milk or dairy products include processed milks, milk beverages, yogurts, lactic acid bacteria beverages, cheeses, ice creams, modified dry milks, creams, and other dairy products.

Examples of oils or fats include butter, margarines, and vegetable oils.

Examples of basic seasonings include soy sauce, Miso, sauces, processed tomato seasonings, Mirin seasonings, and vinegars, and examples of combined seasonings or foods include seasoning mixes, curry rouxes, dipping sources, dressings, noodle soups, spices, and other combined seasonings.

Examples of frozen foods include frozen material foods, frozen semi-prepared foods, and frozen prepared foods.

Examples of confections include caramels, candies, chewing gums, chocolates, cookies, biscuits, cakes, pies, snacks, crackers, Japanese confections, rice confections, bean confections, dessert confections, and other confections.

Examples of beverages include carbonated beverages, natural fruit juices, fruit beverages, refreshing beverages with fruit juice, fruit beverages with pulp, fruit beverage with granules, vegetable beverages, soy milk, soy milk beverages, coffee beverages, tea beverages, powder beverages, concentrated beverages, sport drinks, fortified drinks, alcohol drinks, vinegar-containing beverages, and other favorite drinks.

Examples of other commercial foods than the above include baby foods, Furikake (Japanese rice condiments), and Othazuke Nori (Japanese green tea rice seasonings).

The food or drink product defined in the present technique can be provided or sold as a food or drink product with an indication of a health use.

The "indication" actions include all actions for informing users of the application, including any expression that can evoke or bring to mind that the application falls into the "indication" action of the present technique regardless of the object, content, subject, and medium of the indication.

In addition, the "indication" is preferably provided in such an expression that users can directly recognize the application. Specific examples of indications include: an action to deliver, pass, exhibit for delivery or passing, or import a commodity with respect to a food or drink product with the application written thereon or on a package thereof; and an action to exhibit or distribute advertising, a price list, or a transaction document of a commodity with the application written thereon, or to provide information of such an advertising, a price list, or a transaction document with the application included therein by an electromagnetical method (the Internet or the like).

On the other hand, regarding the content of the indication, the indication is preferably an indication approved by the government or the like (for example, an indication or the like approved under various institutions established by the government and put in a manner based on the approval). The content of the indication is preferably put on a package, a container, a catalog, a pamphlet, an advertising material in the selling site, such as POP, or other documents.

Examples of "indications" also include indications that the commodity is a health food, a functional food, an enteral nutritive food, a food for special uses, a health functional food, a special health food, a nutritive functional food, a functionality-indicated food, a quasi-drug, or the like. Particularly among them, indications approved by Consumer Affairs Agency, for example, indications approved by the institution about special health food or institutions similar thereto are mentioned. Specific examples of the latter indications include an indication that the commodity is a special health food, an indication that the commodity is a conditional special health food, an indication that the commodity may influence on the body structure or function, and an indication about reduction of a disease risk. More specifically, typical examples include an indication that the commodity is a special health food provided in Enforcement Regulations of the Health Promotion Act (Ordinance of the Japanese Ministry of Health, Labor and Welfare No. 86 of April 30, 2003) (especially an indication of a health application) and similar indications thereto.

When acetic acid and/or an acetic acid salt in the present technique is used in a feed, the acetic acid and/or an acetic acid salt may be added to a known feed or may be mixed in raw materials of a feed to produce a new feed.

Examples of raw materials of the feed include grains, such as corn, wheat, barley, and rye; brans, such as wheat bran, barley bran, rice bran, and defatted rice bran; product lees, such as corn gluten meal and corn jam meal; animal-originated feeds, such as skim milk, whey, fish powder, and bone powder; a yeast, such as beer yeast; mineral feeds, such as calcium phosphate and calcium carbonate; oils or fats; amino acids; and sugars. Examples of forms of the feed include feeds for pets (pet foods), livestock feeds, and fish-breeding feeds.

As described above, the present technique can be used in a wide variety of fields of food or drink products, food or drug compositions, functional foods, pharmaceuticals, feeds, and the like.

### Examples

The present technique will be described in more detail below with reference to examples and the like. Note that the examples described below show a typical example of the present technique.

### [Example 1]

As an Alzheimer's disease model mouse, a mouse with excess amyloid β administered in the brain ventricle has been widely used (NPL 6: Takeda et al., Brain Research, 1280, 137-147, 2009.). With reference to NPL 6, Alzheimer's disease model mice were prepared according to the following procedure and the improvement effect of acetic acid was evaluated.

A solution containing 150 mM of sodium acetate was administered with drinking water. Taking the day when the administration was started as Day 1, amyloid β was injected on Day 3. Specifically, an animal was anesthetized by intraperitoneally administering 40 mg/kg of pentobarbital sodium (Tokyo Chemical Industry Co., Ltd.) (amount of liquid administered: 10 mL/kg). After anesthesia, levobupivacaine hydrochloride (Popscaine (registered tradename) 0.25% inj., Maruishi Pharmaceutical Co. Ltd.) was subcutaneously administered (0.1 mL) to the scalp. After anesthesia, hair of a parietal region of the animal was cut and the head was fixed on a brain stereotaxic apparatus.

The scalp was disinfected with ethanol for disinfection and then was cut to expose the cranial bone.

Using a dental drill, a hole for inserting a stainless steel pipe was made in the cranial bone at 1 mm on the lateral side (right) and 0.2 mm on the rear side of bregma, and a stainless steel pipe with an outer diameter of 0.5 mm connected to a silicone tube and a microsyringe was vertically inserted to a depth of 2.5 mm from the bone surface. Into the brain ventricle, 3 µL of an amyloid β solution (200 pmol/3 µL) was injected with a microsyringe pump over 3 minutes. In a pseudo-operation group, 3 µL of PBS was injected with a microsyringe pump over 3 minutes.

After injection, the head was left to stand for 3 minutes with the stainless steel pipe still inserted and the stainless steel pipe was then slowly detached. Then, the stainless steel pipe was removed and the cranial hole was closed with a non-absorbable marrow hemostatic agent (Nestop (registered tradename), Alfresa Pharma Cooperation), and the scalp was sutured.

The administration of acetic acid with drinking water was continued until the end of the action test.

10 ddY male mice were used for each group.

The measurement method of a cognitive function is as follows.

Y-Maze test: a Y-shaped plastic maze (UNICOM Corporation) composed of three arms each having a length of 39.5 cm, a floor width of 4.5 cm, and a wall height of 12 cm and each branching at 120 degree with respect to the other arms was used in the test. After placing the apparatus, the illumination for the floor of the apparatus was adjusted to 20 Lux.

An animal was placed in any one arm of the Y-shaped maze and was allowed to freely sleuth in the maze for 8 minutes. The order of the arms that the animal moved during the measurement period was recorded and the number of the movements to arms was counted and taken as a total entry number.

Next, the number of combinations in each of which different three arms were continuously selected among the total entry number was checked and taken as a spontaneous alternation action number.

A spontaneous alternation action rate was calculated with the following formula. Spontaneous alternation action rate (%) = [spontaneous alternation action number / (total entry number - 2)] × 100

The result is shown in Table 1. It was demonstrated that intake of 150 mM sodium acetate with drinking water improved a cognitive function of Alzheimer disease model mice.

**[Table 1]**

| | Administration subject | Spontaneous alternation action rate (%) | S.E. |
|---|---|---|---|
| Pseud operation group | Physiological saline | 78.8 | 4.6** |
| Amyloid β injection group | Physiological saline | 53.4 | 2.2 |
| Amyloid β injection group | 150mM Sodium acetate | 67.5 | 2** |
| **p<0.01 as compared with (Amyloid β + physiological saline) group | | | |

## Claims

1. A composition comprising acetic acid and/or an acetic acid salt as an active ingredient for use in the treatment of Alzheimer-type dementia.

2. Acetic acid and/or an acetic acid salt for use in the treatment of Alzheimer-type dementia.

3. The composition comprising acetic acid and/or an acetic acid salt as an active ingredient for use according to claim 1, wherein the composition comprising acetic acid and/or an acetic acid salt as an active ingredient is for oral intake, or
the acetic acid and/or an acetic acid salt for use according to claim 2, wherein the acetic acid and/or an acetic acid salt is for oral intake.

4. The composition comprising acetic acid and/or an acetic acid salt as an active ingredient for use according to claim 1 or 3, wherein the composition comprising acetic acid and/or an acetic acid salt as an active ingredient is a food or drink composition, or
the acetic acid and/or an acetic acid salt for use according to claim 2 or 3, wherein the acetic acid and/or an acetic acid salt is comprised in a food or drink composition.

5. The composition comprising acetic acid and/or an acetic acid salt as an active ingredient for use according to any one of claims 1 and 3 to 4, or
the acetic acid and/or an acetic acid salt for use according to any one of claims 2 to 4,
wherein the acetic acid and/or an acetic acid salt is produced by fermentation.

## Patentansprüche

1. Zusammensetzung, die Essigsäure und/oder ein Essigsäuresalz als einen Wirkstoff umfasst, zur Verwendung bei der Behandlung einer Demenz vom Alzheimer-Typ.

2. Essigsäure und/oder ein Essigsäuresalz zur Verwendung bei der Behandlung einer Demenz vom Alzheimer-Typ.

3. Zusammensetzung, die Essigsäure und/oder ein Essigsäuresalz als einen Wirkstoff umfasst, zur Verwendung nach Anspruch 1, wobei die Zusammensetzung, die Essigsäure und/oder ein Essigsäuresalz als einen Wirkstoff umfasst, für die orale Einnahme ist, oder
Essigsäure und/oder ein Essigsäuresalz zur Verwendung nach Anspruch 2, wobei die Essigsäure und/oder ein Essigsäuresalz für die orale Einnahme ist/sind.

4. Zusammensetzung, die Essigsäure und/oder ein Essigsäuresalz als einen Wirkstoff umfasst, zur Verwendung nach Anspruch 1 oder 3, wobei die Zusammensetzung, die die Essigsäure und/oder ein Essigsäuresalz als einen Wirkstoff umfasst, eine Nahrungsmittel- oder Getränkzusammensetzung ist, oder
Essigsäure und/oder ein Essigsäuresalz zur Verwendung nach Anspruch 2 oder 3, wobei die Essigsäure oder ein Essigsäuresalz in einer Nahrungsmittel- oder Getränkzusammensetzung umfasst ist.

5. Zusammensetzung, die Essigsäure und/oder ein Essigsäuresalz als einen Wirkstoff umfasst, zur Verwendung nach einem der Ansprüche 1 und 3 bis 4, oder
Essigsäure und/oder ein Essigsäuresalz zur Verwendung nach einem der Ansprüche 2 bis 4,
wobei die Essigsäure und/oder ein Essigsäuresalz durch Fermentation hergestellt ist/sind.

## Revendications

1. Composition comprenant de l'acide acétique et/ou un sel d'acide acétique en tant que principe actif pour utilisation dans le traitement de la démence de type Alzheimer.

2. Acide acétique et/ou sel d'acide acétique pour utilisation dans le traitement de la démence de type Alzheimer.

3. Composition comprenant de l'acide acétique et/ou un sel d'acide acétique en tant qu'ingrédient actif pour utilisation selon la revendication 1, dans laquelle la composition comprenant de l'acide acétique et/ou un sel d'acide acétique en tant que principe actif est destinée à une administration orale, ou
l'acide acétique et/ou un sel d'acide acétique pour utilisation selon la revendication 2, dans laquelle l'acide acétique et/ou un sel d'acide acétique est destiné à une administration orale.

4. Composition comprenant de l'acide acétique et/ou un sel d'acide acétique en tant que principe actif pour utilisation selon la revendication 1 ou 3, dans laquelle la composition comprenant de l'acide acétique et/ou un sel d'acide acétique en tant que principe actif est une composition alimentaire ou de boisson, ou
l'acide acétique et/ou un sel d'acide acétique pour utilisation selon la revendication 2 ou 3, dans laquelle l'acide acétique et/ou un sel d'acide acétique est compris dans une composition alimentaire ou de boisson.

5. Composition comprenant de l'acide acétique et/ou un sel d'acide acétique en tant que principe actif pour utilisation selon l'une quelconque des revendications 1 et 3 à 4, ou
l'acide acétique et/ou un sel d'acide acétique pour utilisation selon l'une quelconque des revendications 2 à 4,
dans laquelle l'acide acétique et/ou un sel d'acide acétique est produit par fermentation.
